# EUROPEAN PATENT APPLICATION

(11) **EP 4 789 615 A1**
(43) Date of publication of application: **12.08.2026**
(21) Application number: 25156640.2
(22) Date of filing: 07.02.2025
(51) Int. Cl.: A61B 6/04, A61B 6/10, G21F 3/00, A61N 5/10

(54) **FLEXIBLE RADIATION-PROTECTIVE SHIELD FOR AN OPERATING TABLE**

(71) Applicant: Texray AB, 412 92 Göteborg (SE)
(72) Inventor: APELL, Petra, 439 37 Onsala (SE); GELLERSTEDT, Fredrik, 413 09 Göteborg (SE)
(74) Representative: Öberg & Sjöberg AB

(57) **Abstract**

A flexible radiation-protective shield (20) for attachment to an operating table (2), comprising: a support structure (22) having an intended position attached to an operating table (2), preferably to a side (4) of an operating table (2), and a flexible radiation-protective flap (50), comprising a flexible radiation-protective material (40), attached to the support structure (22) and configured to extend above the support structure (22) when the support structure (22) is in the intended position. A system (70) comprising an operating table (2) and at least one flexible radiation-protective shield (20), as well as the use of the flexible radiation-protective shield (20) or the system (70) for protecting clinical staff (12) from radiation during a medical procedure comprising the use of radiation, is also disclosed.

## Description

### Technical field

The technology proposed herein relates to a flexible radiation-protective shield for an operating table, and particularly to a flexible radiation-protective shield comprising a flexible radiation-protective flap extending above a support structure when the support structure is in the intended position attached to an operating table.

### Background

Ionizing radiation, such as X-ray, is commonly used in radiologically guided interventions, such as cardiology, or Interventional Radiology (IR). The clinical staff is exposed to the radiation during the procedures. Current standards and practices are based on the premise that any radiation dose may result in unfavorable health effects and can result in the development of radiation-induced diseases such as a specific type of cancer, glioblastoma. Reducing the radiation exposure of the clinical staff is therefore important.

The radiation can be direct or scattered. In particular, radiation incident on a patient or a part of a patient will scatter, both at the point where the radiation impinges on the patient but also throughout the body of the patient. Further scattering occurs as the radiation impinges on the operating table.

During the radiologically guided intervention, or procedure, the patient is typically placed on an operating table. A source of radiation is provided above or underneath the operating table. The source of radiation may be positioned in various angular projections in relation to the patient. One or more radiation-protective drapes or coverings may be placed on the patient to prevent a major part of the radiation scattered in the body of the patient from irradiating out of the patient to reach the clinical staff. During the radiologically guided intervention the clinical staff typically stand along the left long side of the operating table for access to the interventional vascular entry points such as femoralis or radialis.

Additionally, a radiation-protective curtain may be positioned below a long side of the operating table for preventing radiation emanating under the operation table, e.g. from radiation leakage from the source of radiation or from scattering, from reaching the clinical staff that typically stand along the long side of the operating table.

Further, it is known to use separate radiation-protective shields, for example top shield (Kenex), which are manually fastened to a table skirt or curtain at the side of the operating table and extend above the surface of the operating table and any mattress placed on the operating table surface to stop radiation scattered horizontally by the patient from reaching the clinical staff. Separate radiation-protective shields however require additional work to use and may be bulky to store and/or handle.

### Objects of the proposed technology

The proposed technology aims at increasing the protection against radiation while at the same time obviating the need for attaching and detaching radiation-protective shields to the sides of the operating table. It is a further object of the proposed technology to provide a flexible radiation-protective shield for attachment to an operating table.

### Summary

A first aspect of the proposed technology concerns a flexible radiation-protective shield for attachment to an operating table, comprising:
- a support structure having an intended position attached to an operating table, preferably to a side of an operating table, and
- a flexible radiation-protective flap, comprising a radiation-protective material, attached to the support structure and configured to extend above the support structure when the support structure is in the intended position.

The inclusion of a flexible radiation-protective flap provides radiation protection along and above the sides of the operating table. In particular, by comprising a flexible radiation-protective flap, the radiation-protective shield can both extend above the side of the operating table to provide radiation protection when the patient is laying on the operating table, and bend to accommodate the patient when the patient is transferred, or lifted, e.g. in a supine or prone position, onto and off the operating table. There is thus no need to detach and reattach the radiation-protective shield from the operating table for these procedures as was the case with prior art rigid radiation-protective shields. This improves ergonomics and efficiency of the handling of the patient.

Accordingly, the shield does not include hinges, joints, etc to provide flexibility.

It is specified that the radiation-protective shield is flexible. Expressed differently, the radiation-protective shield is capable of flexing, or deforming, when subjected to force, such as force applied on the shield from any part of a patient contacting the shield during transferral, or movement, of a patient onto, and off, the operating table. The radiation-protective shield is thus pliable. The radiation-protective shield may be at least partially elastic, or resilient, whereby the radiation-protective shield essentially, or substantially, resumes an initial shape and configuration, in particular one in which it extends un an upwards direction, e.g. vertically, once a force deforming the shield in a sideways direction, e.g. horizontally, is no longer applied to the shield. This ensures that the radiation-protective shield provides protection against radiation travelling in an at least partially horizontal direction from a patient laying on the operating table once the patient has been placed on the operating table. Preferably, the flexible radiation-protective shield is configured to be bendable by the application of a force of at least 1 N to 100 N, such as at least 5 N to 50 N, and less than 500 N, such as less than 200 N, e.g. preferably a force in the range of 1 N to 100 N, applied to the upper edge of the flexible radiation-protective shield, i.e. to the upper horizontal edge of the flexible radiation-protective flap. At these forces, bending occurs at least at the lower horizontal edge of the flexible radiation-protective flap, and typically throughout the flexible radiation-protective flap. The extent of bending possible for the flexible radiation-protective flap depends on the position of the upper horizontal edge in relation to the operating table and any mattress provided on the operating table when the flexible radiation-protective flap is in its normal position, i.e. extending substantially vertical up from the support structure. The extent of bending, in particular towards the operating table, i.e. when a patient is transferred onto the operating table, is thus typically in the range of at least 15°, such as at least 30°, preferably at least 45°, more preferably at least 60° compared to vertical when the support structure is in the intended position. Generally, the extent of bending away from the operating table, i.e. when the patient is transferred off the operating table, is the same as for bending in the opposite direction. In the absence of a force acting on the flap, the flap will recover to an essentially upright position

It is specified that the shield is radiation-protective. Expressed differently, the covering protects against, or attenuates, ionizing radiation. Expressed differently, the shield may be an X-ray protective shield. The shield, in particular the flap, may comprise a radiation attenuating material, in particular an X-ray attenuating material. The shield, particular the flap, may be arranged or configured to attenuate X-ray radiation from a radiation source having a tube voltage of at least with energies of at least 40 kV, preferably at least 60 kV, and at the most 150 kV, preferably at the most 110 kV. For example, the covering may be arranged or configured to attenuate X-ray radiation from a radiation source having a tube voltage with energies at least in the range of 40-150 kV, such as 60-110 kV. The covering may be arranged or configured to provide at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, in particular at least 0.50 mm, Pb equivalence, at any tube voltage in the range 60-110 kV. Pb equivalence is to be measured according to Inverse Broad Beam Geometry as described in IEC 61331-1:2014. The shield, in particular the flap, may be non-transparent or non-translucent to optical, or visible, light.

The flexible radiation-protective shield may be dimensioned to extend along one or several sides of the operating table.

Thus, the flexible radiation-protective shield may extend along more than one side of the operating table, i.e. it may bend around one or more corners of the operating table.

Preferably, the flexible radiation-protective shield may be dimensioned to extend along a part of the length of the side of the operating table, i.e. the long side, whereby preferably a plurality, such as two or three, of flexible radiation-protective shields are provided adjacent to each other along the side of the operating table. Generally, the flexible radiation-protective shield may have a width, or length, of at least 0.2 m, preferably at least 0.6 m, more preferably at least 0.8 m. The maximum length of the flexible radiation-protective shield may correspond to the circumference of the operating table, i.e. be several meters. Preferably however, the length of the flexible radiation-protective shield is 1 m or less. Further, generally, the flexible radiation-protective shield may have a height of at least 10 cm, preferably at least 15 cm, more preferably at least 20 cm, such as at least 25 cm or at least 30 cm. The flexible radiation-protective shield may have a height of at the most 50 cm, preferably at the most 45 cm, more preferably at the most 40 cm.

It is specified that the flexible radiation-protective shield is for attachment to an operating table. Expressed differently, the flexible radiation-protective shield is suitable for attachment to an operating table, preferably to the side of an operating table. The flexible radiation-protective shield may be rendered suitable for attachment to the operating table, preferably to the side of the operating table by providing an attachment structure on the support structure, or by forming the support structure to comprise an attachment structure, the attachment structure being configured for engaging the operating table, preferably the side of an operating table, or for engaging a corresponding attachment structure provided on the operating table, preferably on or in the side of the operating table.

The attachment structure on the support structure may comprise any of hooks, fasteners (screws, rivets), magnets, or a weld. Preferably the attachment structure on the support structure comprises a profile having a cross section engaging a corresponding cross section on a profile provided on or in operating table or on or in the side of the operating table.

The side of an operating table encompasses a lateral vertical end surface of the operating table, as well as the upper and lower horizontal surfaces of the operating table surface and its underside, respectively, along the side of the operating table. In particular, the horizontal area extending between an edge of the surface of the operating table and a position within ¼ to 1/8, preferably within 1/16, of the width of the operating table from that edge may further be encompassed by the side of an operating table. Preferably the side of the operating table is limited to a lateral vertical end surface of the operating table. The side of the table may preferably be a long side of the operating table, i.e. a side of the operating table extending along the largest dimension of the operating table. The operating table may for example have a length of at least 1 m, preferably at least 1.5 m. The operating table may have a width of at least 0.4 m, preferably at least 0.6 m. The upper surface of the operating table may be provided at least 0.6 m, preferably at least 0.8 m above a floor surface upon which the operating table is placed. The operating table may be configured to provide selectable lifting or lowering of the operating table. An attachment structure may be provided on or in the side of the operating table for engaging an attachment structure provided on, or by, the support structure.

It is specified that the support structure has an intended position attached to an operating table, preferably to a side of an operating table. Expressed differently, the support structure is configured to be attachable to the operating table, preferably to the side of an operating table, and, when so attached, has an intended position. Preferably the support structure is only attachable to the operating table when placed in the intended position.

Preferably the support structure comprises an attachment structure engaging a corresponding attachment structure provided on the operating table, preferably on or in the side of the operating table, as described above. The attachment structure may comprise a profile such as rail with a corresponding attachment structure comprising a groove engaging and retaining the rail. The rail may for example be T-shaped, or L-shaped, in cross section and the groove may correspondingly be C-shaped, or J-shaped in cross section, or vice versa. Alternatively, the attachment structure may comprise discrete attachment point where fasteners such as screws or rivets attach the support structure to the operating table or the side of the operating table. Additionally, the support structure may be attached to the operating table or the side of the operating table by being integrally formed with the operating table or the side of the operating table, or by being welded or glued to the operating table or the side of the operating table.

Additionally, an attachment structure comprised by the holder may comprise a clamping arrangement to clamp the side of the operating table or to clamp a structure such as a rail, profile, or sphere arranged on the operating table or in or at the side or the operating table. Additionally, an attachment structure comprised by the holder may comprise a plug or pin for being retained by a hole arranged on the operating table or in or at the side of the operating table.

An attachment structure associated with the holder may be formed integrally with the holder or may be attached to the holder.

The support structure may be elongated, such as an arm, beam, or elongated profile. The support structure may thus comprise an arm, beam or profile having a length corresponding to the width or length of the flexible radiation-protective shield. Alternatively, the support structure may comprise a plurality of support structure portions or section.

The radiation-protective flap, as well as the flexible radiation-protective material, is flexible and radiation-protective as described above for the flexible radiation-protective shield. The flexible radiation-protective flap may comprise or consist of the flexible radiation-protective material. The flexible radiation-protective flap may have a length, or width, corresponding to the length, or width, of the support structure. The flexible radiation-protective flap may have a height corresponding to the height of the flexible radiation-protective shield. In particular, the flexible radiation-protective flap may have a height positioning an upper edge of the radiation-protective flap at, or above, the surface of the operating table, or the surface of a mattress provided on the surface of the operating table, when the support structure is in the intended position. Preferably the flexible radiation-protective flap may have a height of at least 5 cm, preferably at least 10 cm, more preferably at least 15 cm. Preferably the flexible radiation-protective flap may have a height at the most 30 cm, preferably at the most 25 cm, more preferably at the most 30 cm. Preferably the flexible radiation-protective flap has a height of 5-30 cm, more preferably 5-25 cm, most preferably 10-20 cm. The flexible radiation-protective flap may have a width, or length, of at least 0.2 m, preferably at least 0.6 m, more preferably or at least 0.8 m. The maximum length of the flexible radiation-protective flap may correspond to the circumference of the operating table, i.e. be several meters. Preferably however, the length of the flexible radiation-protective flap is 1 m or less.

The flexible radiation-protective flap may have a thickness of at least 0,5 mm, preferably at least 1 mm. Preferably, the flexible radiation-protective flap has a thickness of at the most 10 mm, preferably at the most 5 mm.

The flexible radiation-protective flap may thus extend from a lower edge, or lower horizontal edge, attached to, or in, the support structure, to an upper edge, or upper horizontal edge. The distance between the lower edge and the upper edge defines a height of the flexible radiation-protective flap and further defines the extension of the flexible radiation-protective flap above the support structure. Additionally, the flexible radiation-protective flap may extend from a first side edge, or first vertical edge, to a second side edge, or second vertical edge. The distance between the first and second vertical edges defines a length of the flexible radiation-protective flap. The lower and upper edges are preferably parallel. The first and second side edges are preferably parallel. The flexible radiation-protective flap may in particular be rectangular.

The flexible radiation-protective material may comprise, or consist of, a permeable fabric, or fibrous material. The permeable fabric may comprise filaments. The filaments may be arranged in a regular pattern. The filaments may extend in a uniform direction. The uniform direction may be aligned with, or parallel with, the extension of the flexible radiation-protective flap above the support structure when the support structure is in the intended position. Worded differently, the uniform direction may be parallel to the vertical direction when the support structure is in the intended position. This increases the stiffness and longevity of the flexible radiation-protective shield. Each filament may be of a solid composition that comprises a carrier substance and a radiopaque substance. For example, the solid composition may be a polymeric composite material. The filaments may be arranged in a regular pattern. It is understood that each filament may be a monofilament. It is further understood that the carrier substance and the radiopaque substance may be mixed, and that the radiopaque substance may be evenly distributed within the carrier substance. The carrier substance may comprise at least one polymer and the radiopaque substance may comprise at least one metal. For example, the polymer may be a thermoplastic polymer such as a polyvinyl chloride or a polyolefin and the metals may, as a powder, be as an element, oxide or alloy of for example Lead, Barium, Antimony, Bismuth, or Tungsten (Wolfram), and any combination thereof.

The fabric may be a woven fabric with the filaments forming the weft of the fabric. It is understood that the fabric has a warp, for example of polyester. More specifically, the first material may be any of the radiation-protective material described in WO2014163574A1. The fabric contributes to higher longevity of the flexible radiation-protective shield.

Alternatively, the flexible radiation-protective material may be, or comprise, an impermeable sheet. The impermeable sheet may be of a solid composition that comprises a carrier substance and a radiopaque substance. For example, the solid composition may be a polymeric composite material. It is further understood that the carrier substance and the radiopaque substance may be mixed, and that the radiopaque substance may be evenly distributed within the carrier substance. The carrier substance may comprise at least one polymer and the radiopaque substance may comprise at least one metal.

It is understood that the flexible radiation-protective material may be composed of several layers, for example two layers of fabric or two impermeable sheets. As specified above for the flexible radiation-protective shield, the flexible radiation-protective material may have at least 0.05, mm, preferably at least 0.10 mm, more preferably at least 0.25 mm, most preferably at least 0.50 mm, and at the most 1.00 mm, preferably at the most 0.75 mm, more preferably at the most 0.50 mm Pb equivalence at any tube voltage in the range 60-110 kV. For example, the covering may be arranged or configured to provide 0.05 to 1.00 mm, preferably 0.10 to 1.00 mm, more preferably 0.25 to 1.00 mm, such as 0.25 to 0.75 mm or 0.30 to 0.60 mm, most preferably at least 0.50 mm Pb equivalence at any tube voltage in the range 60-110 kV.

It is specified that the flexible radiation-protective flap is attached to the support structure. Expressed differently, the support structure carries, or positions, the flexible radiation-protective flap when the support structure is in the intended position. The flexible radiation-protective flap may for example be attached to the support structure using any of adhesive, fasteners such as screws or rivets

Preferably, the flexible radiation-protective flap may be attached to the support structure by being sandwiched between parts, such as halves, of the support structure. Alternatively, or additionally, the flexible radiation-protective flap may be attached to the support structure by any of screws, rivets, and adhesive.

It is specified that the flexible radiation-protective flap is configured to extend above the support structure when the support structure is in the intended position. Expressed differently, the flexible radiation-protective flap extends perpendicularly, or vertically upwards, from the support structure in the intended position.

Preferably the flexible radiation-protective flap has a height of at least 5 cm, preferably at least 10 cm, more preferably at least 15 cm, above the support structure.

This is preferable in that it further decreases radiation scattered from the patient or originating under the operating table.

The flexible radiation-protective flap preferably has a height of at the most 30 cm, preferably at the most 25 cm, more preferably at the most 20 cm, above the support structure.

The flexible radiation-protective flap may for example extend up to the upper surface of the operating table, or up to the upper surface of a mattress lying on the operating table, when the support structure is in the intended position.

Preferably the flexible radiation-protective flap has a length of least 0.2 m, more preferably at least 0.6 m, most preferably at least 0.8 m.

For ease of handling, it is generally preferable to use a plurality of flexible radiation-protective shields attached one after the other to the operating table, preferably to the side of the operating table instead of a single flexible radiation-protective shield extending along the whole side of the operating table, although the latter is possible.

Preferably the radiation-protective flap has a length of at the most 3 m, more preferably at the most 1.8 m, most preferably at the most 1.5 m. Suitable lengths for the radiation-protective flap are 0.2 to 0.6 m, 0.6 to 0.8 m, 0.6 to 1 m, and 0.8 to 1.2m. A plurality of flexible radiation-protective shields provides for modularity when using the flexible radiation-protective shields with different operating tables having different lengths, and for different clinical procedures having different requirements for protection from radiation. The flexible radiation-protective shields may be attached to the operating table along one or more sides of the operating table.

Preferably the flexible radiation-protective material comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

These are suitable flexible radiation-protective materials. These materials are described in more detail above.

When the filaments extend in a uniform direction, the uniform direction may preferably be aligned with, or parallel with, a vertical direction when the support structure is in the intended position. This increases the stiffness of the flexible radiation-protective flap thus maintaining it extending upwards above the support structure except when deflected, bent or deformed by a patient during transferral of the patient onto or off the operating table.

Preferably the flexible radiation-protective material comprises a permeable fabric that comprises filaments as described above. Such a permeable fabric has high tolerance, i.e. is more durable, to being bent, or deformed, when the flexible radiation-protective shield is bent when a patient is transferred onto and off the operating table. This ensures longevity of the flexible radiation-protective shield.

Preferably the support structure comprises an elongated arm, beam or profile, the support structure preferably having a length equal to or shorter than the length of the flexible radiation-protective flap.

This is advantageous in that it provides improved and continuous support for the flexible radiation-protective flap along the length of the flap. Furthermore, the elongated arm, beam or profile in itself will serve to block part of the radiation originating from below the operating table, as well as comprising, or providing at attachment point for, an attachment structure for attaching the support structure to the side of the operating table.

When the support structure comprises an elongated arm or elongated beam, then the elongated arm or beam may be provided with at least one attachment structure. When the support structure comprises an elongated profile, one side of the elongated profile may be configured as an attachment structure.

When the support structure has a length shorter than the length of the flexible radiation-protective flap, the length of the support structure is preferably at 1-10 cm, more preferably 1-5 cm shorter than the flexible radiation-protective flap. Preferably then the distances between the opposite ends of the support structure and the corresponding opposite vertical edges of the flexible radiation-protective flap are equal.

The elongated arm, beam or profile may be made from metal, such as aluminium or steel.

Preferably the support structure comprises an elongated groove or rail configured to, in the intended position, slide onto, and be retained by, an elongated rail or groove provided on or in the side of the operating table.

This provides a secure attachment of the support structure to the operating table. The groove and rail may provide additional protection from radiation originating from below the operating table. The groove may for example be C shaped and the rail T-shaped.

Preferably the support structure comprises an inner support structure facing the side of the operating table in the intended position, and an outer support structure facing away from the side of the operating table in the intended position, wherein the flexible radiation-protective flap is attached to the support structure by being sandwiched between the inner support structure and the outer support structure.

This is advantageous in that it provides a secure attachment of the flexible radiation-protective flap to the support structure. This is also advantageous in that it allows attaching medical equipment to the outer support structure.

In this configuration it is the inner support structure that is attached to operating table, preferably the side of the operating table.

Having an inner support structure and an outer support structure is especially advantageous when the support structure is elongated, e.g. as an elongated arm, beam or profile. Accordingly, the inner and outer support structures may thus comprise elongated arms, beams or profiles.

Preferably the sides of the inner and outer support structures that face the flexible radiation-protective flap are flat to ensure a good contact with the flexible radiation-protective material. Additionally, or alternatively, an adhesive may be provided between the inner and outer support structures and the flexible radiation-protective material to further secure the attachment of the flexible radiation-protective flap to the support structure.

Preferably the outer support structure comprises an elongated groove or rail, preferably wherein the outer support structure comprises an elongated groove or rail corresponding to an elongated groove or rail provided on or in the side of the operating table.

This is advantageous in that it provides for attaching further medical equipment to the operating table via the flexible radiation-protective shield. It further provides for attaching more than one layer of flexible radiation-protective shields onto, preferably a side of, an operating table.

Preferably the outer support structure is attached to the inner support structure by fasteners passing through the outer support structure, through the flexible radiation-protective material, and into the inner support structure.

This is advantageous in that it makes the radiation-protective shield easy to assemble.

The fasteners are preferably screws or rivets. A bore, or hole, may be provided in the outer support structure and a corresponding bore, or hole, may be provided in the inner support structure, for the fasteners to pass through. The bores or holes in the outer support structure may be throughgoing and the bores or holes in the inner support structure may be blind bores or hols, or vice versa. Throughgoing bores or holes may be nonthreaded or threaded, while blind bores or holes may be threaded.

Preferably the flexible radiation-protective shield further comprises:
- a flexible radiation-protective curtain, comprising a flexible radiation-protective material, attached to the support structure and configured to extend below the support structure when the support structure is in the intended position.

This is advantageous in that it further protects the clinical staff from radiation originating or scattered below the operating table.

The curtain is flexible and radiation-protective as described above for the flexible radiation-protective shield. The flexible radiation-protective curtain may comprise or consist of the flexible radiation-protective material as described above.

Preferably the flexible radiation-protective material of the flexible radiation-protective curtain is the same as the flexible radiation-protective material of the flexible radiation-protective flap.

The flexible radiation-protective curtain may be attached to the support structure as described for the flexible radiation-protective flap described above.

It is specified that the flexible radiation-protective curtain is configured to extend below the support structure when the support structure is in the intended position. Expressed differently, the flexible radiation-protective curtain extends vertically downwards, or perpendicularly and opposite the radiation-protective flap, from the support structure in the intended position.

The flexible radiation-protective curtain may extend from an upper edge, or upper horizontal edge, to a lower edge, or lower horizontal edge, the distance between the upper edge and the lower edge defining the height of the flexible radiation-protective curtain, i.e. the distance that the flexible radiation-protective curtain extends below the support structure in the intended position. The flexible radiation-protective curtain may further extend from a first edge, or first vertical edge, to a second edge, or second vertical edge, the distance between the first and second edges defining the length of the flexible radiation-protective curtain.

Preferably the flexible radiation-protective curtain has a height of at least 50 cm, more preferably at least 60 cm, most preferably at least 75 cm below the support structure.

The flexible radiation-protective curtain may for example extend down to the floor on which the operating table stands, when the support structure is in the intended position.

This is advantageous in that it increases protection from radiation originating below the operating table.

Preferably the flexible radiation-protective curtain has a length corresponding to the length of the flexible radiation-protective flap.

This ensures a uniform extent of radiation protection both below and above the support structure.

Preferably the radiation-protective flap and the radiation-protective curtain are formed from the same piece of flexible radiation-protective material.

Expressed differently, the same piece of flexible radiation-protective material extends both above and below the support structure in the intended position. This is advantageous in that a single, or unbroken, piece of flexible radiation-protective material making up both the flexible radiation-protective flap and the flexible radiation-protective curtain decreases the risk of radiation passing through the flexible radiation-protective shield.

A second aspect of the proposed technology concerns a system comprising an operating table and at least one flexible radiation-protective shield according to the first aspect of the proposed technology.

The operating table and the flexible radiation-protective shield are as described above.

A third aspect of the proposed technology concerns the use of the flexible radiation-protective shield according to the first aspect of the proposed technology, or the system according to the second aspect of the proposed technology, for protecting clinical staff from radiation during a medical intervention comprising the use of radiation.

The medical intervention may comprise a radiologically guided intervention, such as cardiology, or Interventional Radiology (IR). The medical intervention, or medical procedure, may comprise diagnostic and/or surgical procedures. The medical intervention may be a fluoroscopic, or fluoroscopy, assisted intervention.

The clinical staff may comprise any personnel present during the medical intervention.

The third aspect of the proposed technology may alternatively be considered a method of protecting clinical staff from radiation during a medical intervention comprising the use of radiation, comprising the steps of:
- providing:
   - the flexible radiation-protective shield according to the first aspect of the proposed technology and an operating table, or
   - the system according to the second aspect of the proposed technology, and
- attaching the flexible radiation-protective shield to the operating table, preferably to the side of the operating table.

### Brief description of the drawings

A more complete understanding of the abovementioned and other features and advantages of the technology proposed herein will be apparent from the following detailed description of preferred embodiments in conjunction with the appended drawings, wherein:
**Fig. 1** shows a perspective exploded view of an embodiment of a flexible radiation-protective shield.
**Fig. 2** shows a closeup perspective exploded view of the embodiment of the flexible radiation-protective shield shown in Fig. 1.
**Fig. 3** shows a perspective view and side of two flexible radiation-protective shields as shown in Fig. 1 with support structures in the intended position attached to, here the side of, an operating table.
**Figs. 4a and 4b** show a patient being transferred onto an operating table to which the embodiment of the flexible radiation-protective shield shown in Fig. 1 has been attached.
**Fig. 5** shows medical equipment being carried by an outer support structure.
**Fig. 6** shows three flexible radiation-protective shields generally as shown in Fig. 1 with support structures in the intended position attached to the side of an operating table.

### Detailed description

In the figures and the below description, the same reference numerals are used for the same or related features. One or more ` added to a reference number indicates that the feature so designated is a variant of the feature bearing the reference number without the '. A subscript number indicates that the features so references is a further one of the feature bearing the reference number without the subscript number.

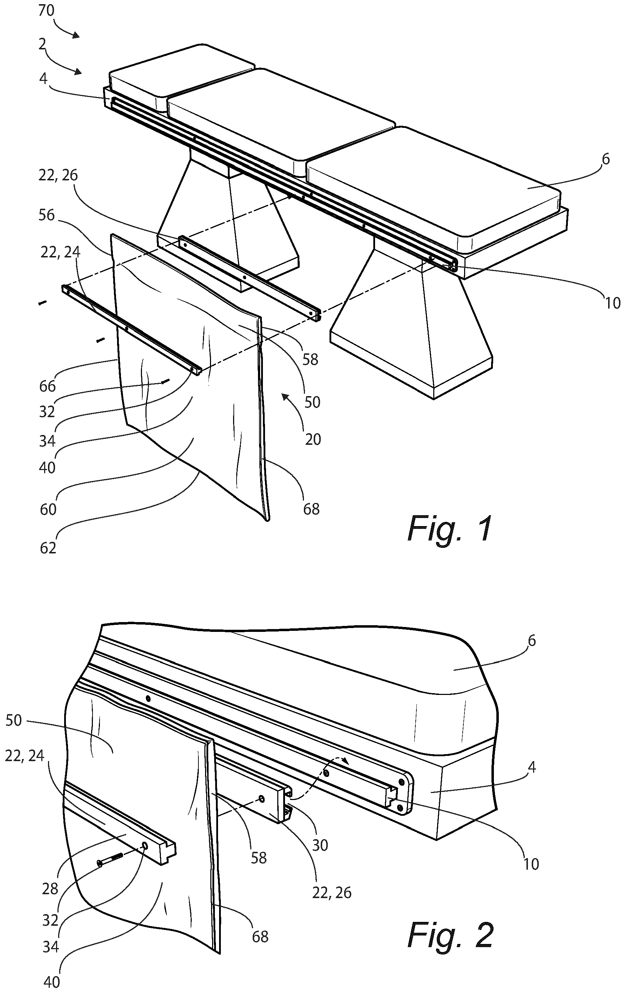

A perspective exploded view, and a closeup perspective exploded view, of an embodiment of a flexible radiation-protective shield is shown in **Fig. 1** and **Fig. 2****,** respectively.

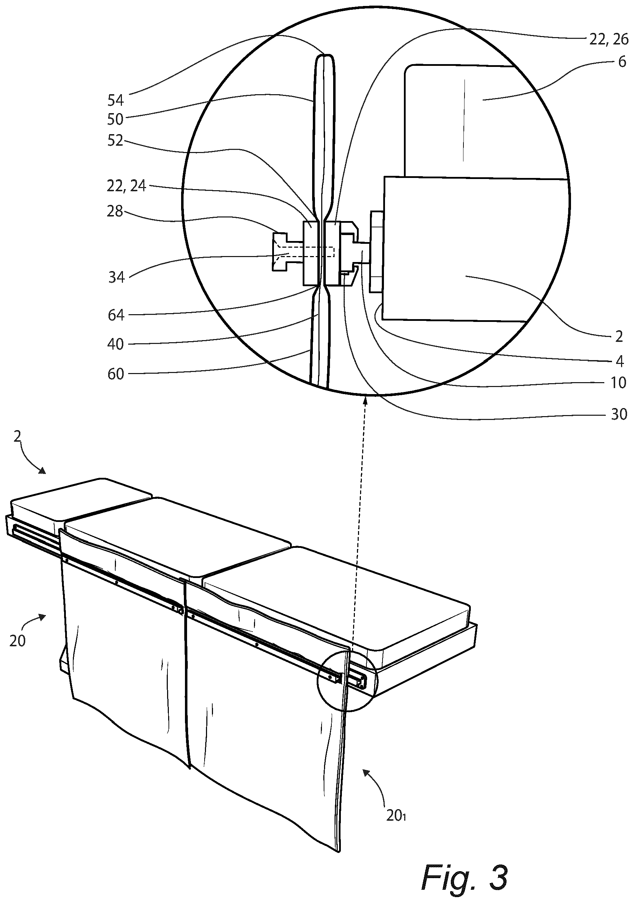

Fig 1 thus shows an operating table 2 having a side 4 and carrying a mattress 6 onto which a patient 8 (not shown in figs. 1 and 2) may be placed during a clinical procedure. The side 4 of the operating table 2 is provided with an elongated rail 10 representing an attachment structure, e.g. for attaching medical equipment to the side 4 of the operating table 2, or, as in this case, attaching the flexible radiation-protective shield 20. Flexible radiation-protective shield 20 comprises a support structure 22 in the form of an elongated profile, made up of an outer support structure 24 formed as an elongated profile and an inner support structure 26 also formed as an elongated profile. The outer and inner support structures 24 and 26 are here made from aluminium. As noted, the outer support structure 24 further carries an elongated rail 28 having a similar cross section as the elongated rail 10 on the side 4 of the operating table 2. The inner support structure 26 comprises an elongated groove 30 configured to slide onto, and be retained by, the elongated rail 10 on the side 4 of the operating table 2. Between the outer and inner support structures 24 and 26, which are joined together by machine screws 32 in bores 34, is provided a flexible radiation-protective material 40 defining a flexible radiation-protective flap 50 above the support structure 22. The flexible radiation-protective flap 50 comprises lower horizontal edge 52 at the support structure 22 and an opposite upper horizontal edge 54, see Fig. 3, as well as a first vertical edge 56 and an opposite second vertical edge 58. The height of the flexible radiation-protective flap 50, i.e. the vertical distance between the lower horizontal edge 52 and the upper horizontal edge 54 is here 20 cm which provides that the upper horizontal edge 54 reaches the upper surface of the mattress 6 on the operating table 2. The length of the flexible radiation-protective flap 50, i.e. the horizontal distance between the first vertical edge 56 and the second vertical edge 58 is here 0.8 m.

Below the support structure 22 the flexible radiation-protective material 40 defines a flexible radiation-protective curtain 60 having a lower horizontal edge 62 close to the floor on which the operating table 2 is positioned, as well as an opposite upper horizontal edge 64 at the support structure 22, see also Fig. 3. A first vertical edge 66 is further provided opposite to a second vertical edge 68.

The height of the flexible radiation-protective curtain 60, i.e. the vertical distance between the lower horizontal edge 62 and the upper horizontal edge 64 is here 80 cm. The length of the flexible radiation-protective curtain 60, i.e. the horizontal distance between the first vertical edge 66 and the second vertical edge 68 is here 0.8 m.

The length of the outer and inner support structures 24, 26, i.e. the length of the support structure 22, is here slightly shorter than the length of flexible radiation-protective flap 50 and curtain 60 at 76 cm.

The flexible radiation-protective material 50 is a permeable fabric that comprises monofilaments arranged in a regular pattern and extending in a uniform direction, for example as described in WO2014163574A1. Each filament is solid composition that comprises a carrier substance of polyolefin and radiopaque substance of Antimony and Bismuth powder that are uniformly mixed. The fabric is a woven fabric with the filaments forming the weft of the cloth and with a warp of polyester. The flexible radiation-protective material 40 can attenuate X-ray radiation from a radiation source having a tube voltage of between 40 to 150 kV. The flexible radiation-protective material 40 used in the embodiments of the flexible radiation-protective shield shown in the figures has at least 0.50 mm Pb equivalence at any tube voltage in the range 60-110 kV.

The operating table 2 and the flexible radiation-protective shield 20 constitute a system 70.

As seen in greater detail in **Fig. 3****,** the flexible radiation-protective material 40 is pressed and sandwiched between the outer and inner support structure 24, 26, and thus safely attached to the support structure 22. Fig. 3 further shows the interaction between the elongated rail 10 on the side 4 of the operating table 2 and the elongated groove 30, as well as the cross-sectional profile of the elongated rail 28 on the outer support structure 24 with the outline of the bores 34 in the outer support structure 24 and in the inner support structure 26. Fig. 3 further shows how a plurality of flexible radiation-protective shields 20, 20₁ may be attached to the side 4 of the operating table 2 so as to provide continuous protection along at least part of the side 4. Here it is also discernible that, using a support structure 22 having a length that is smaller than the length of the flexible radiation-protective flap and curtain 50, 60, the flexible radiation-protective shields 20 may be brought sufficiently together such that the flexible radiation-protective flap and curtain 50, 60 of one flexible radiation-protective shield 20 overlaps with the flexible radiation-protective flap and curtain 50, 60 of the other flexible radiation-protective shield 20₁ so as to prevent radiation from passing between the flexible radiation-protective shields 20.

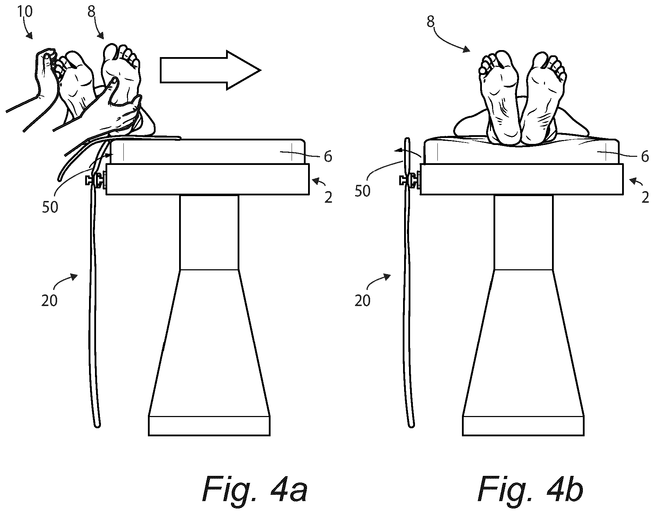

**Figs. 4a** and **4b** show a patient 8 being transferred onto the operating table 2 to lay down on the mattress 6. To facilitate the transfer, the patient is placed on a support sheet 14 which may be tugged or pulled to move the patient 8 on to the mattress 6, and, once the patient 8 is in place on the mattress 6, may be removed as has been done in Fig. 4b. As seen in Fig. 4a, during the transferral of the patient 8 onto the mattress 6, as illustrated by hands of clinical staff 12, the flexible radiation-protective flap 50 can be bent inwards to simplify the transfer. Once the patient has been placed onto the mattress 6, the flexible radiation-protective flap 50 spontaneously resumes its normal position extending vertically above the support structure 22. Similarly, although not shown in Figs. 4a and 4b, the flexible radiation-protective flap 50 can be bent outwards to facilitate the removal of the patient from the mattress 6 and the operating table 2. The usefulness of the flexibility of the flexible radiation-protective flap 50 increases even further when its height is increased since even a flexible radiation-protective flap 50 being higher than the one shown in Figs 4a and 4b will bend inwards and outwards, as needed, during transfer of the patient 8 while extending substantially vertically above the support structure 22 during the clinical procedure.

Additionally, as seen, the flexible radiation-protective curtain 60, by being flexible, allows the operating table 2 to be lowered if desired, whereby the flexible radiation-protective curtain 60 bends against the floor upon which the operating table is placed, thus ensuring reliable radiation protection.

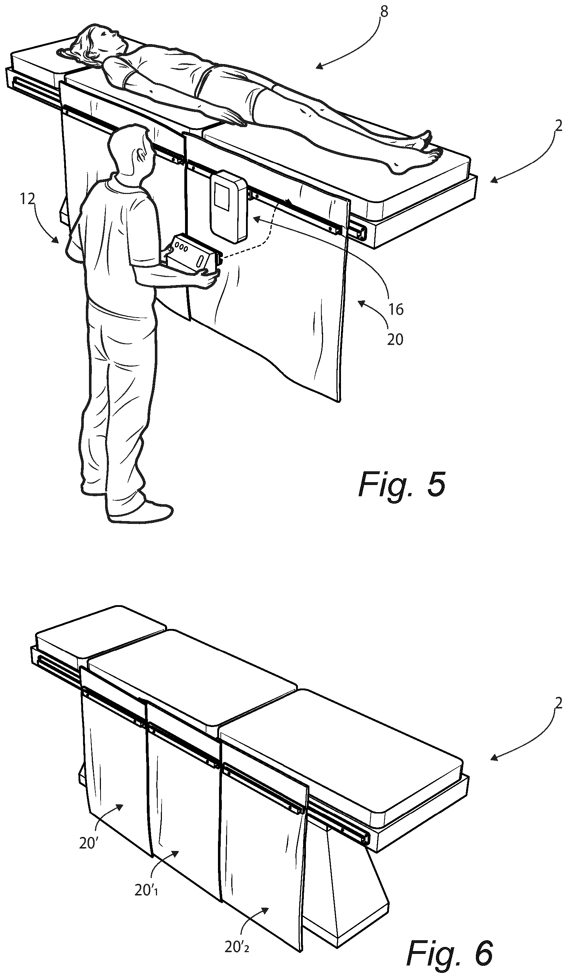

**Fig. 5** shows medical equipment 16 being carried by the elongated rail 28 provided on the outer support structure 24 of the flexible radiation-protective shield 20. Accordingly, by using an outer support structure 24 having an elongated rail 28 corresponding to the elongated rail 10 of the operating table 2, the possibility of attaching and carrying medical equipment to and by the operating table 2 remains despite the attachment of the flexible radiation-protective shield 20 to the side 4 of the operating table 2. As is further clear from Fig. 5 is that clinical staff 12, who during a clinical procedure stands along the side 4 of the operating table 2, is protected from radiation originating below the operating table 2, including radiation emitted upwards from below the operating table 2, as well as from radiation scattered from the patient in the plane of the mattress 6. For completeness, it is noted that the patient 8 may additionally be covered by a radiation-protective drape or blanket, not shown, which further reduces radiation scattered from the patient. Likewise, the clinical staff 12 would, during a clinical procedure, carry additional wearable radiation-protective shields such as radiation-protective garments.

**Fig. 6** shows three flexible radiation-protective shields 20'. 20'₁, 20'₂ of the same type as radiation-protective shield 20 but being shorter, i.e. having less length, with support structures in the intended position attached to an operating table. As noted with reference to fig. 3, the flexible radiation-protective flaps and curtains 50, 60 overlap so as to prevent radiation from passing between the flexible radiation-protective shields.

### Item list

2 operating table
4 side of operating table
6 mattress
8 patient
10 elongated rail
12 clinical staff
14 support sheet
16 medical equipment
20, 20' flexible radiation-protective shield
22 support structure
24 outer support structure
26 inner support structure
28 elongated rail
30 elongated groove
32 screws
34 bores
40 flexible radiation protective material
50 flexible radiation-protective flap
52 lower horizontal edge
54 upper horizontal edge
56 first vertical edge
58 second vertical edge
60 flexible radiation-protective curtain
62 lower horizontal edge
64 upper horizontal edge
66 first vertical edge
68 second vertical edge
70 system

## Claims

1. A flexible radiation-protective shield (20) for attachment to an operating table (2), comprising:
- a support structure (22) having an intended position attached to an operating table (2), preferably to a side (4) of an operating table (2), and
- a flexible radiation-protective flap (50), comprising a flexible radiation-protective material (40), attached to the support structure (22) and configured to extend above the support structure (22) when the support structure (22) is in the intended position.

2. The flexible radiation-protective shield (20) according to claim 1, wherein the flexible radiation-protective flap (50) has a height of at least 5 cm, such as at least 10 cm, more preferably at least 15 cm, above the support structure (22) when the support structure (22) is in the intended position.

3. The flexible radiation-protective shield (20) according to any preceding claim, wherein the flexible radiation-protective flap (50) has a length of least 0.2 m, preferably at least 0.6 m or at least 0.8 m.

4. The flexible radiation-protective shield (20) according to any preceding claim, wherein the flexible radiation-protective material (40) comprises:
a permeable fabric that comprises filaments, each filament being of a solid composition that comprises a carrier substance and a radiopaque substance, and the fabric is a woven fabric with the filaments forming the weft of the fabric, or
an impermeable sheet formed from a solid composition comprising a carrier substance and a radiopaque substance.

5. The flexible radiation-protective shield (20) according to any preceding claim, wherein the support structure (22) comprises an elongated arm, beam or profile (22), the support structure (22) preferably having a length equal to or shorter than the length of the flexible radiation-protective flap (50).

6. The flexible radiation-protective shield (20) according to any preceding claim, wherein the support structure (22) comprises an elongated groove or rail (30) configured to, in the intended position, slide onto, and be retained by, an elongated rail or groove (10) provided on or in the side (4) of the operating table (2) .

7. The flexible radiation-protective shield (20) according to any preceding claim, wherein the support structure (22) comprises an inner support structure (26) facing the side (4) of the operating table (2) in the intended position, and an outer support structure (24) facing away from the side (4) of the operating table (2) in the intended position, wherein the flexible radiation-protective flap (50) is attached to the support structure (22) by being sandwiched between the inner support structure (26) and the outer support structure (24).

8. The flexible radiation-protective shield (20) according to claim 7, wherein the outer support structure (24) comprises an elongated groove or rail (28), preferably wherein the outer support structure (24) comprises an elongated groove or rail (28) corresponding to an elongated groove or rail (10) provided on or in the side (4) of the operating table (2).

9. The flexible radiation-protective shield (20) according to any of claims 7-8, wherein the outer support structure (24) is attached to the inner support structure (26) by fasteners (32) passing through the outer support structure (24), through the flexible radiation-protective material (40), and into the inner support structure (26).

10. The flexible radiation-protective shield (20) according to any preceding claim, further comprising:
- a flexible radiation-protective curtain (60), comprising a flexible radiation-protective material (40), attached to the support structure (22) and configured to extend below the support structure (22) when the support structure (22) is in the intended position.

11. The flexible radiation-protective shield (20) according to claim 10, wherein the flexible radiation-protective curtain (60) has a height of at least 50 cm, preferably at least 60 cm, more preferably at least 75 cm below the support structure (22) when the support structure (22) is in the intended position.

12. The flexible radiation-protective shield (20) according to any of the claims 10-11, wherein the flexible radiation-protective curtain (60) has a length corresponding to the length of the flexible radiation-protective flap (50).

13. The flexible radiation-protective shield (20) according to any of the claims 10-12, wherein the flexible radiation-protective flap (50) and the flexible radiation-protective curtain (60) are formed from the same piece of flexible radiation-protective material (40).

14. A system (70) comprising an operating table (2) and at least one flexible radiation-protective shield (20) according to any of the preceding claims.

15. Use of the flexible radiation-protective shield (20) according to any of the claims 1-13, or the system (70) according to claim 14, for protecting clinical staff (12) from radiation during a medical intervention comprising the use of radiation.
